# EUROPEAN PATENT APPLICATION

(11) **EP 3 437 583 A1**
(43) Date of publication of application: **06.02.2019**
(21) Application number: 18187069.2
(22) Date of filing: 02.08.2018
(51) Int. Cl.: A61B 90/30

(54) **MEDICAL SHADOWLESS LAMP**

(30) Priority: 03.08.2017 JP 2017151164
(71) Applicant: Sum Tech Innovations Co., Ltd., Tsuyama-shi, Okayama 708-0872 (JP)
(72) Inventor: TAKAMOTO, Tomohisa, Tsuyama-shi, Okayama 708-0886 (JP); INAO, Kazushiko, Tsuyama-shi, Okayama 708-0014 (JP); TSUJI, Takeshi, Yashio-shi, Saitama 340-0822 (JP)
(74) Representative: Weickmann & Weickmann PartmbB

(57) **Abstract**

A medical shadowless lamp is constituted by disposing a plurality of irradiation units in an irradiation body, disposing a plurality of LED units in the irradiation unit, disposing LED chips which emit three primary colors of red (R), green (G), and blue (B) in the LED unit, and disposing an LED chip which emits white (W) at the same time. Alternatively, a color LED chip in which an LED which emits white (W), an LED which emits red (R), an LED which emits green (G), and an LED which emits blue (B) are integrated may be disposed. In addition, an irradiation angle can be changed by allowing a peripheral irradiation unit to be freely inclined at a predetermined angle with respect to a central irradiation unit.

## Description

### BACKGROUND

### Technical Field

The present invention relates to a medical shadowless lamp using an LED as a light source.

### Related Art

Since areas of a patient are shadowed during surgery or treatment due to fingers of doctors who perform the surgery or treatment or surgeon tools or treatment tools used, a shadowless lamp as an illumination device which does not generate a shadow during the surgery or treatment is used so that a doctor does not have trouble in performing the surgery or treatment.

Conventionally, an incandescent bulb, a krypton bulb, a halogen bulb, and the like have been frequently used as the light source for the shadowless lamp, but there is a problem in that if these light sources generating high heat are used, a body temperature rises, and thus a burden on a patient is increased and surgical areas or the like are dried early, such that surgery or the like is hindered.

For this reason, in recent years, as the light source of the shadowless lamp, an LED that can be expected to generate low heat has been used. The light source disclosed in JP 2006-156074A as such a medical shadowless lamp has been known.

### SUMMARY

However, since the medical shadowless lamp with the conventional LED adopts an LED which emits white, when the surgical tools or the like are irradiated with light from a diffused reflection or the like, and thus it becomes difficult to see affected parts or the like, such that surgery or the like is hindered.

Also, due to bleeding at affected parts or the like and a difference in pigments in eyes for each race, it sometimes becomes somewhat difficult for a doctor to see affected parts when a doctor performs surgery or treatment.

In addition, a color temperature of the white LED causes the doctor to be less calm and causes patient's anxiety.

In addition, in the case of a medical shadowless lamp with the conventional LED, when a doctor performs surgery or treatment, illuminance of light irradiated on affected parts or the like is not sufficient, such that it sometimes becomes difficult for the doctor to perform the surgery or the like.

In addition, in the case of performing surgery such as an incision of a surface layer of an affected part, high illuminance may not be required but in the case of performing surgery such as an incision of an inner part of the affected part, illuminance needs to be sufficiently high. However, the conventional medical shadowless lamp cannot adjust illuminance as well as luminous intensity.

The present invention has been made in view of such conventional problems, and it is an object of the present invention to provide a medical shadowless lamp with no problem that it becomes difficult to see affected parts due to diffused reflection of light irradiated on surgical tools during surgery or treatment and therefore surgery or the like is hindered, it becomes somewhat difficult to see the affected parts due to bleeding at the affected parts or the like and a difference in pigments in eyes, and a doctor may become less calm and a patient may feel anxious.

Another object of the present invention is to provide a medical shadowless lamp capable of easily performing surgery by sufficiently securing illuminance of light irradiated to affected parts when a doctor performs surgery or treatment, and appropriately adjusting the illuminance of light irradiated to the affected parts without consuming wasteful power under a situation during surgery or treatment.

To achieve the above object, according to the present invention, there is provided a medical shadowless lamp, wherein a plurality of irradiation units are disposed in an irradiation body, LED units are disposed in the irradiation units, and LED chips which emit three primary colors of red (R), green (G), and blue (B) are disposed in the LED units.

The LED chips which emit three primary colors of red (R), green (G), and blue (B) may be disposed in the LED units at appropriate intervals in a circumferential direction.

The LED chips which emit the three primary colors of red (R), green (G), and blue (B) and an LED chip which emits white (W) may be simultaneously disposed in the LED units.

Particularly, a color LED chip in which an LED which emits white (W), an LED which emits red (R), an LED which emits green (G), and an LED which emits blue (B) are integrated is preferably disposed in the LED unit.

Further, in the medical shadowless lamp of the present invention, an irradiation body is constituted by a central irradiation unit disposed in a central part thereof and a plurality of peripheral irradiation units disposed in a peripheral part thereof, and the peripheral irradiation unit is freely inclined at a predetermined angle with respect to the central irradiation unit.

Here, the peripheral irradiation units are preferably disposed at equal intervals around the central irradiation unit.

Furthermore, according to the medical shadowless lamp of the present invention, the irradiation body is constituted by the plurality of irradiation units divided at equal intervals in a circumferential direction, and in each irradiation unit, the LED unit is radially disposed from a center.

In the medical shadowless lamp of the present invention, since the LED chips which emits the three primary colors of red (R), green (G), and blue (B) are disposed in the LED unit disposed in the irradiation unit, the light irradiated on surgical tools or the like suffers from the diffused reflection or the like when a doctor performs surgery or treatment, and therefore it becomes difficult to see the affected parts or the like, thereby not hindering the surgery or the like.

In addition, there is no problem in that due to bleeding at the affected parts or the like and the difference in pigments of eyes when a doctor performs surgery or treatment, it becomes somewhat difficult to see the affected parts or the like and a doctor may become less calm and a patient may feel anxious.

In addition, in the medical shadowless lamp of the present invention, since the peripheral irradiation unit is freely inclined at a predetermined angle with respect to the central irradiation unit, the irradiation angle of the peripheral irradiation unit is changed to condense light, such that it is possible to sufficiently secure the illuminance of light irradiated on the affected parts or the like and to facilitate the surgery or the like.

In addition, the irradiation angle of the peripheral irradiation unit is changed to be able to appropriately adjust the illuminance of light irradiated on the affected parts or the like during the surgery or treatment, such that it is possible to prevent the wasteful power consumption.

### BRIEF DESCRIPTION OF DRAWINGS

FIG. 1 is a perspective view of a floor-mounted type medical shadowless lamp according to the present invention;
FIG. 2 is a perspective view of a ceiling-mounted type medical shadowless lamp according to the present invention;
FIG. 3 is a front view of an irradiation body of a medical shadowless lamp according to the present invention;
FIG. 4 is a front view of an irradiation unit of the medical shadowless lamp according to the present invention;
FIG. 5 is a front view of an LED unit of the medical shadowless lamp according to the present invention;
FIG. 6 is a perspective view of the ceiling-mounted type medical shadowless lamp according to the present invention;
FIG. 7 is a front view of the irradiation body of the medical shadowless lamp shown in FIG. 6;
FIG. 8 is an explanatory diagram of a simple model for evaluating illuminance of a targeted irradiation surface in a case in which a peripheral irradiation unit is inclined at a predetermined angle in the medical shadowless lamp shown in FIG. 6;
FIGS. 9A and 9B are light distribution diagrams showing light distribution patterns of a central irradiation unit and the peripheral irradiation unit in the medical shadowless lamp shown in FIG. 6, wherein FIG. 9A is a light distribution diagram showing a wide angle light distribution pattern and FIG. 9B is a light distribution diagram showing a narrow angle light distribution pattern;
FIGS. 10A and 10B are explanatory views showing a case in which the peripheral irradiation unit is not inclined with respect to a central irradiation body and the light distribution pattern is set as wide angle light distribution;
FIG. 11 is an illuminance distribution diagram showing the result of simulating the illuminance of the targeted irradiation surface in the case shown in FIGS. 10A and 10B;
FIGS. 12A and 12B are explanatory views showing in the case in which the light distribution pattern is set as the wide angle light distribution in the case in which the peripheral irradiation unit is not inclined at a predetermined angle with respect to the central irradiation body;
FIG. 13 is an illuminance distribution diagram showing the result of simulating the illuminance of the targeted irradiation surface in the case shown in FIGS. 12A and 12B;
FIGS. 14A and 14B are explanatory views showing a case in which the peripheral irradiation unit is inclined at a predetermined angle with respect to the central irradiation body and the light distribution pattern is set as narrow angle light distribution;
FIG. 15 is an illuminance distribution diagram showing the result of simulating the illuminance of the targeted irradiation surface in the case shown in FIGS. 14A and 14B;
FIG. 16 is a front view of an LED unit of a medical shadowless lamp according to another embodiment of the present invention;
FIG. 17 is a front view of a color LED chip disposed in the LED unit shown in FIG. 16;
FIG. 18 is a graph showing spectral characteristics of white, red, green and blue LEDs mounted on the color LED chip shown in FIG. 17;
FIG. 19 is an explanatory view showing a case in which in the medical shadowless lamp according to the present invention, an irradiation unit in which an irradiation body having a disk shape is divided into six parts in a circumferential direction is configured and in each irradiation unit, the LED unit is radially disposed from the center;
FIG. 20 is an explanatory view showing an irradiation state while each irradiation unit is held horizontally in the medical shadowless lamp shown in FIG. 19;
FIG. 21 is an explanatory view showing an irradiation state while each irradiation unit is inclined from a horizon in the medical shadowless lamp shown in FIG. 19; and
FIG. 22 is an explanatory view showing an irradiation state while in the medical shadowless lamp shown in FIG. 19, each irradiation unit is inclined by 10° from a horizon.

### DETAILED DESCRIPTION

Hereinafter, a medical shadowless lamp of the present invention will be described in detail with reference to the drawings.

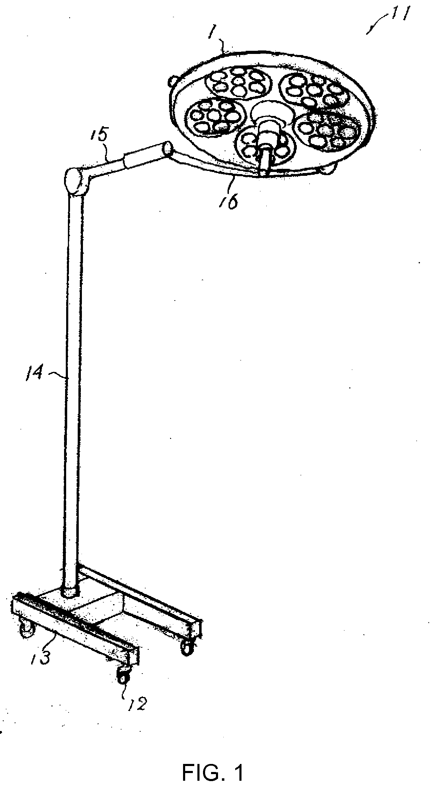

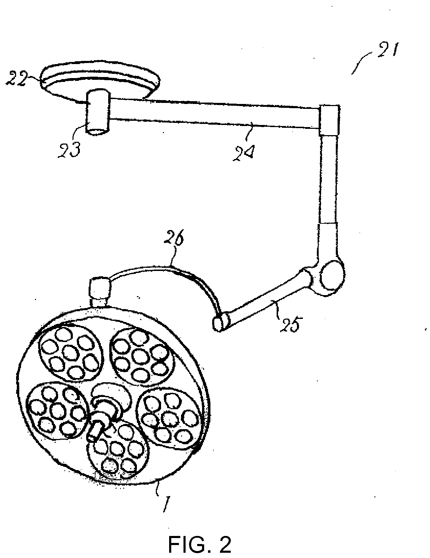

FIG. 1 is a perspective view of a floor-mounted type medical shadowless lamp according to the present invention, and FIG. 2 is a perspective view of a ceiling-mounted type medical shadowless lamp according to the present invention.

The medical shadowless lamp of the present invention can be applied to a floor-mounted medical shadowless lamp 11 as shown in FIG. 1.

In the medical shadowless lamp 11, a column 14 stands from a leg 13 equipped with wheels 12, 12,..., a supporting rod 15 is connected to an upper end of the column 14 to be rotatable in a vertical direction, an arm 16 is connected to a tip of the supporting rod 15 to be rotatable by 360°, and an irradiation body 1 is connected to the tip of the arm 16.

The medical shadowless lamp of the present invention can be applied to a ceiling-mounted type medical shadowless lamp 21 as shown in FIG. 2.

In a medical shadowless lamp 21, a column 23 hangs down from a mounting body 22, a support rod 24 is connected to a circumferential surface of the column 23 to be rotatable by 360° in a horizontal direction, a support rod 25 is connected to a lower end of the support rod 24 to be rotatable in a vertical direction, an arm 26 is connected to a tip of the support rod 25 to be rotatable by 360°, and the irradiation body 1 is connected to a tip of the arm 26.

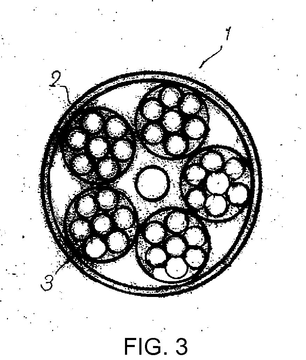

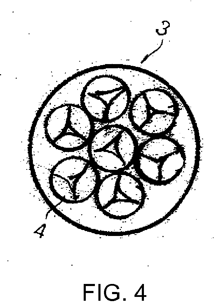

As shown in FIG. 3, in the irradiation body 1, a plurality of irradiation units 3, 3,... are disposed on a bottom surface of a disk-shaped housing 2 at equal intervals in a circumferential direction, and as shown in FIG. 4, a plurality of LED units 4 are disposed at a central part and a peripheral part on an inner side of the irradiation units 3.

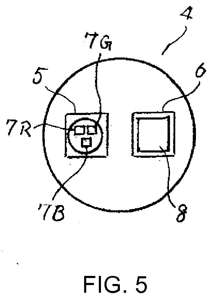

As shown in FIG. 5, an LED chip 5 and an LED chip 6 are disposed inside the LED unit 4.

Here, the LED chip 5 is provided with LEDs 7R, 7G, and 7B which emit three primary colors of red (R), green (G) and blue (B), and the LED chip 6 is provided with an LED 8 which emits white (W).

In order to control a ray of light emitted from the LED chips 5 and 6, it is possible to adopt a refraction method using a lens or a reflection method using a reflector, but the lens method is significantly affected by chromatic aberration, and therefore it is preferable to use the refractor method.

That is, it is preferable that an outer periphery of the LED unit 4 is surrounded by the reflector to control the ray of light.

The medical shadowless lamp of the present invention includes the LEDs 7R, 7G, and 7B which emit the three primary colors of red (R), green (G), and blue (B) or includes the LED 8 which emits white (W) in addition to the LEDs 7R, 7G, and 7B.

Here, the LEDs 7R, 7G, and 7B may be provided separately in the plurality of LED units 4, or may be simultaneously disposed in a single LED unit 4 as described above.

In addition, each LED unit 4 is freely inclined at a predetermined angle by inclining the surrounding reflector, such that the LED irradiation light is converged inwardly and is diffused outwardly.

The medical shadowless lamps 11 and 21 of the present invention have the above-mentioned configuration, and the column 14 is gripped and moved or the support rod 24 is gripped and rotated to move the irradiation body 1 to an appropriate position around an operating table, an examination table or the like.

If an appropriate voltage is applied to the LED chip 5 which emits the three primary colors of red (R), green (G), and blue (B) to appropriately adjust an emission amount of the LEDs 7R, 7G, and 7B of red (R), green (G), and blue (B), there is no problem in that it becomes difficult to see affected parts due to diffused reflection or the like of light irradiated on surgical tools or the like during surgery or treatment, and therefore the surgery or the like is hindered.

In addition, there is no problem in that due to bleeding at the affected parts or the like and the difference in pigments of eyes during surgery or treatment, it becomes somewhat difficult to see the affected parts or the like and a doctor may become less calm and a patient may feel anxious.

In addition, by appropriately mixing the LEDs 7R, 7G, and 7B and irradiating light from the plurality of LED units 4, 4,..., it is possible to change colors of light from monochrome and mixed color to white and appropriately irradiate a color of light.

In addition, if the emission amount is changed for each of the LEDs 7R, 7G, and 7B, it is possible to appropriately form irradiation light having different colors and color tones and form a color environment corresponding to a patient's preference.

If both of the LED chip 5 that emits the three primary colors of red (R), green (G) and blue (B) and the LED chip 6 that emits white (W) are disposed in the LED unit, it is possible to more efficiently select white light or mixed light.

As described above, according to the medical shadowless lamp 1 of the present invention, there is no problem in that it becomes difficult to see affected parts due to diffused reflection or the like of light irradiated on surgical tools or the like during surgery or treatment, and therefore the surgery or the like is not hindered.

In addition, there is no problem in that due to bleeding at the affected parts or the like and the difference in pigments of eyes during surgery or treatment, it becomes somewhat difficult to see the affected parts or the like and a doctor may become less calm and a patient may feel anxious.

Next, a medical shadowless lamp according to another embodiment of the present invention will be described.

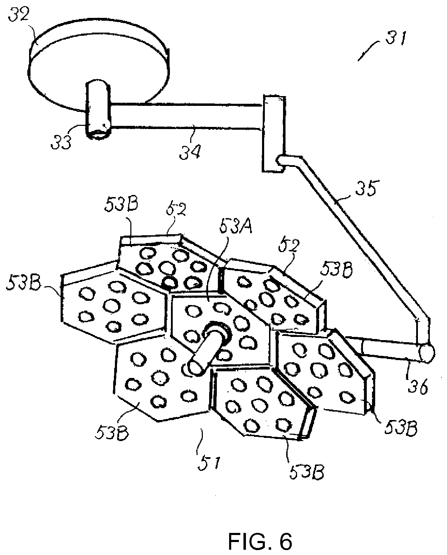

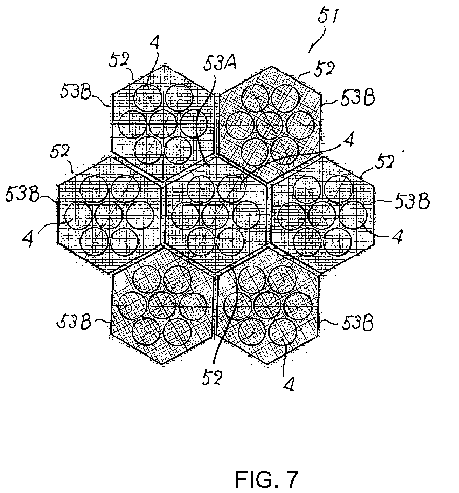

FIG. 6 is a perspective view of a ceiling-mounted type medical shadowless lamp according to the present invention, and FIG. 7 is a front view of an irradiation body according to the present invention.

In a medical shadowless lamp 31, as shown in FIG. 6, a column 33 hangs down from a mounting body 32, a support rod 34 is connected to a circumferential surface of the column 33 to be rotatable by 360° in a horizontal direction, a support rod 35 is connected to a lower end of the support rod 34 to be rotatable in a vertical direction, an arm 36 is connected to a tip of the support rod 35 to be rotatable by 360°, and an irradiation body 51 is connected to a tip of the arm 36.

The medical shadowless lamp 31 of the present invention is characterized by the configuration of the irradiation body 51, and as shown in FIGS. 6 and 7, the irradiation body 51 is constituted by a plurality of irradiation units 53 which are disposed on a bottom surface of a hexagonal housing 52, but a plurality of peripheral irradiation units 53B, 53B,... which are disposed in a peripheral part of the irradiation body 51 are freely inclined at a predetermined angle with respect to a central irradiation unit 53A which is disposed in a central part of the irradiation body 51.

Here, the peripheral irradiation units 53B, 53B,... are disposed at equal intervals around the central irradiation unit 53A.

By doing so, when the peripheral irradiation units 53B, 53B,... are simultaneously inclined at a predetermined angle, the irradiation light can be condensed in a line symmetry manner.

Like the medical shadowless lamps 11 and 21 described above, as shown in FIG. 4, the plurality of LED units 4 are disposed in a central part and a peripheral part on an inner side of the irradiation units 53A and 53B, as shown in FIG. 5, the LED chip 5 and the LED chip 6 are disposed inside the LED unit 4.

As a means for inclining the peripheral irradiation unit 53B, a link mechanism for supporting the respective peripheral irradiation units 53B is disposed on the central irradiation unit 53A, and the link mechanism is operated by an air cylinder, such that the respective peripheral irradiation units 53B may be simultaneously inclined at the same angle.

In addition, a support member for supporting the respective peripheral irradiation units 53B is disposed on the central irradiation unit 53A and the support member is operated by a stepping motor, such that the respective peripheral irradiation units 53B may be simultaneously inclined at the same angle.

Apart from that, various types of means for inclining the peripheral irradiation unit 53B can be considered. However, since it is necessary to position the peripheral irradiation unit 53B at a predetermined angle with high accuracy by smoothly inclining the peripheral irradiation unit 53B, in particular, it is preferable to operate the support member by the stepping motor or a DC motor with a feedback control.

Next, according to the medical shadowless lamp 31 of the present invention, in order to confirm whether light is condensed by changing the illumination angle of the peripheral irradiation unit 53B to sufficiently secure the illuminance of light irradiated on affected parts, a simulation was implemented by a computer.

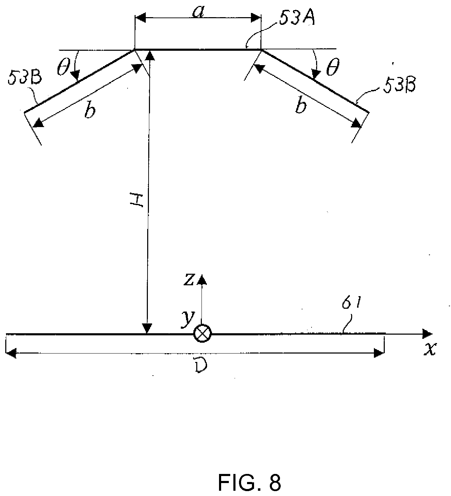

In order to implement the simulation, as shown in FIG. 8, when the peripheral irradiation units 53B and 53B are inclined at a predetermined angle, a simple model for evaluating illuminance of a targeted irradiation surface 61 is considered.

Here, x and y indicate a horizontal direction, and z indicates a vertical direction. If the central irradiation unit 53A of the irradiation body 51 has a square of a × a, the peripheral irradiation unit 53B is a square of b × b, the inclination angle of the peripheral irradiation unit 53B with respect to the central irradiation unit 53A is set to be 0.

In addition, the irradiation surface 61 to be evaluated is an area having a square of D × D, and a height of the central irradiation unit 53A of the irradiation body 51 with respect to the irradiation surface 61 is set to be H.

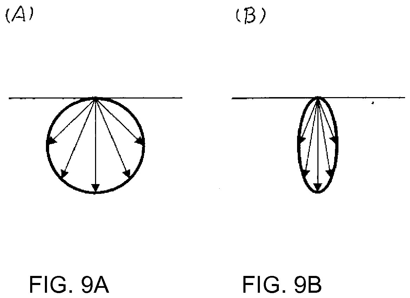

In addition, as light distribution patterns of the central irradiation unit 53A and the peripheral irradiation unit 53B, a wide angle light distribution pattern shown in FIG. 9A or a narrow angle light distribution shown in FIG. 9B is selected.

Here, the light distribution represents how to spread the emitted light, and the light distribution pattern is represented by a light distribution diagram showing intensities of light in up, down, left, and right directions.

For example, the wide angle light distribution is defined as a case in which a range of half of downward luminosity is more than 30° and less than 90°, and the narrow angle light distribution is defined as a case in which a range of half of downward luminosity is less than 150°.

### [Example 1]

A width a of the central irradiation unit 53A of the irradiation body 51 was set to be 200 × 200 mm, a width b of the peripheral irradiation unit 53B was set to be 500 × 500 mm, a width D of the irradiation surface 61 was set to be 1200 × 1200 mm, and a height H of the central irradiation unit 53A of the irradiation body 51 was set to be 1200 mm.

In addition, the central irradiation unit 53A and the peripheral irradiation unit 53B were provided with the plurality of LED units 4, and a luminous flux (luminosity) of the respective irradiation unit was set to be 1001 m.

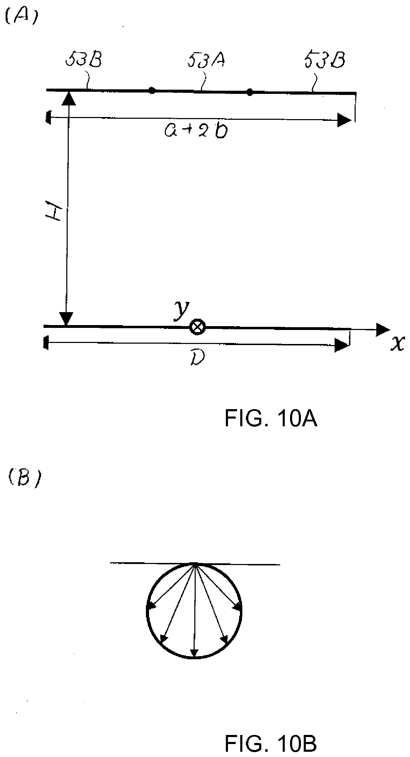

Then, the wide angle light distribution was selected without inclining the peripheral irradiation units 53B and 53B as shown in FIG. 10A, that is, by setting the inclination angle θ to be 0° and setting the light distribution patterns of the unit 53 A and the peripheral irradiation unit 53B as shown in FIG. 10B.

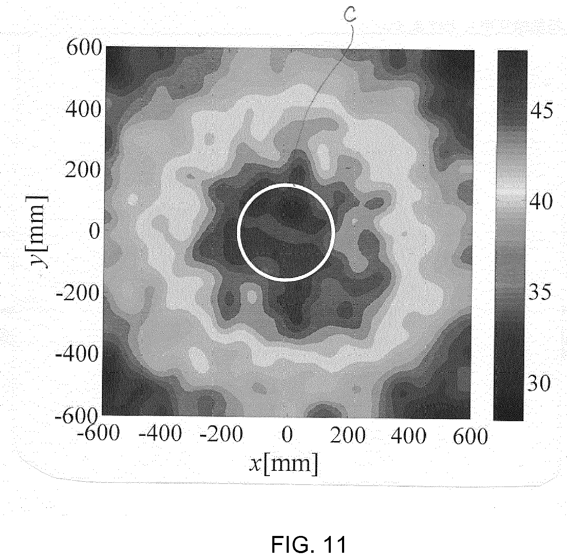

In this case, the illuminance distribution on the irradiation surface 61 was as shown in FIG. 11. An average value of the illuminance in region C having a diameter of 200 mm from a center point of the irradiation surface 61 was 46.4241x.

### [Example 2]

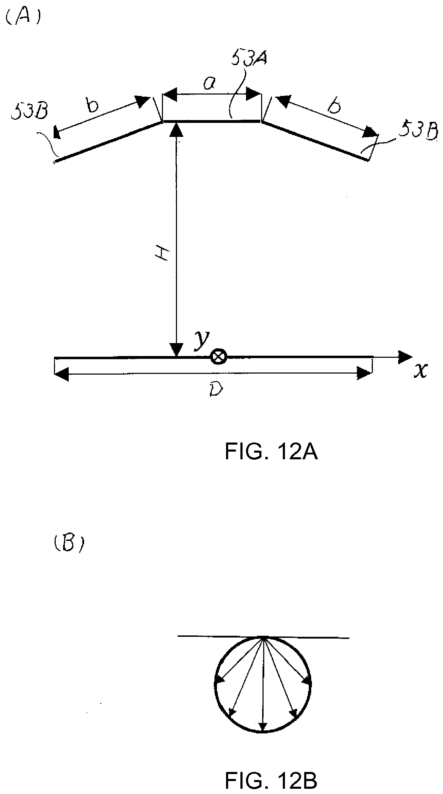

Next, the wide angle light distribution was selected by inclining the peripheral irradiation units 53B and 53B to set the inclination angle θ to be 20° as shown in FIG. 12A and setting the light distribution patterns of the central irradiation unit 53A and the peripheral irradiation unit 53B as shown in FIG. 12B. Other conditions were the same as in Example 1.

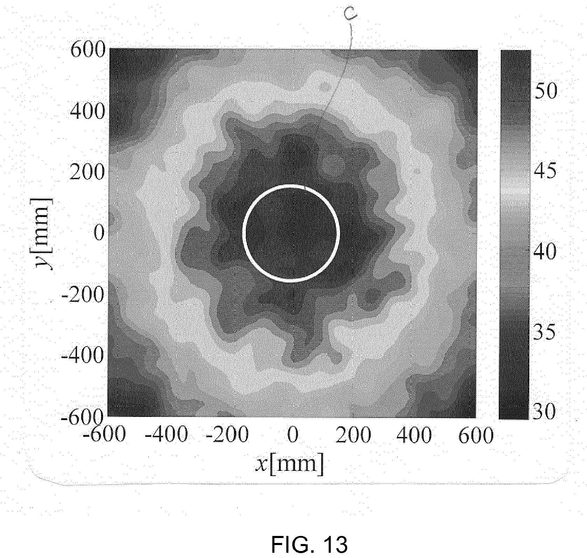

In this case, the illuminance distribution on the irradiation surface 61 was as shown in FIG. 13. The average value of the illuminance in the region C having a diameter of 200 mm from the center point of the irradiation surface 61 was 51.6751x.

Here, if a ratio of average illuminance when the inclination angle is 20° to an average illuminance when the inclination angle is 0° is defined as a condensing rate, the value of the condensing rate was 1.10.

### [Example 3]

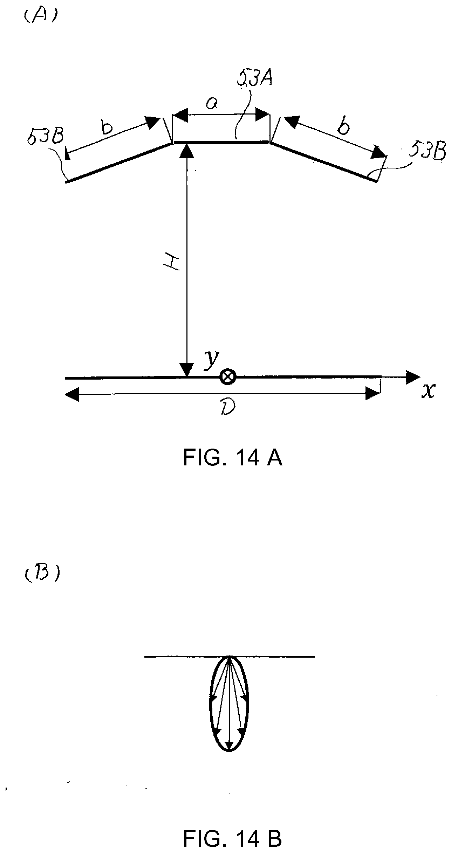

Next, the narrow angle light distribution was selected by inclining the peripheral irradiation units 53B and 53B to set the inclination angle θ to be 20° as shown in FIG. 14A and setting the light distribution patterns of the central irradiation unit 53A and the peripheral irradiation unit 53B as shown in FIG. 14B. Other conditions were the same as in Example 1.

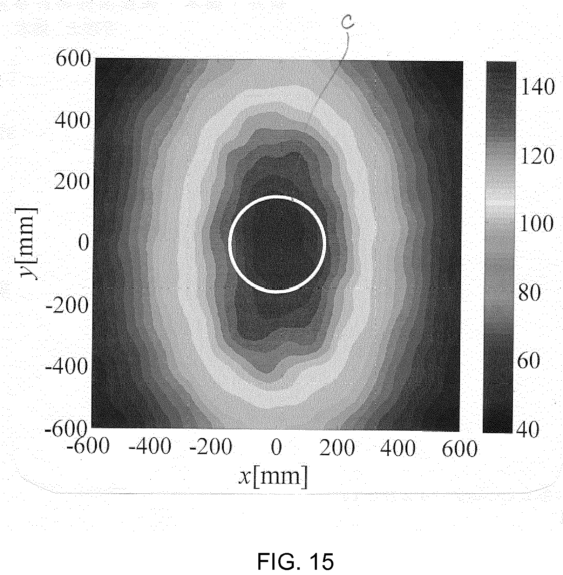

In this case, the illuminance distribution on the irradiation surface 61 was as shown in FIG. 15. The average value of the illuminance in the region C having a diameter of 200 mm from the center point of the irradiation surface 61 was 147.8571x.

Here, the ratio of the average illuminance when the inclination angle is 20° to the average illuminance when the inclination angle is 0°, that is, the value of the condensing rate was 3.20.

According to the medical shadowless lamp 31 of the present invention, by inclining the peripheral irradiation units 53B and 53B at a predetermined angle with respect to the central irradiation unit 53A based on the above simulation results, it was found that the irradiation light from the peripheral irradiation units 53B and 53B can be condensed to sufficiently secure the illuminance of the light irradiated on the affected parts or the like.

In addition, if the narrow angle light distribution is selected as the light distribution patterns of the central irradiation unit 53A and the peripheral irradiation unit 53B, it is possible to further condense the irradiation light to further increase the illuminance of the light irradiated on the affected parts or the like.

In the above description, the ceiling-mounted type medical shadowless lamp 31 has been described, but it goes without saying that it can also be applied to a floor-mounted medical shadowless lamp.

In addition, although the irradiation body 51 is constituted by the plurality of irradiation units 53 disposed on the bottom surface of the hexagonal housing 52, it is not limited to the hexagonal shape, but the housing having various shapes such as a circular shape or a rectangular shape can be selected.

As described above, according to the medical shadowless lamp of the present invention, since the light is condensed by changing the irradiation angle of the peripheral irradiation unit to sufficiently secure the illuminance of the light irradiated on the affected parts or the like, the surgery or the like can be easily performed.

In addition, since the irradiation angle of the peripheral irradiation unit is changed to be able to appropriately adjust the illuminance of light irradiated on the affected parts or the like during the surgery or treatment, wasteful power consumption is prevented, such that energy can be saved.

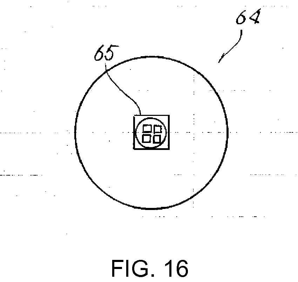

Instead of the LED unit 4 as shown in FIG. 5, an LED unit 64 as shown in FIG. 16 may be adopted.

As shown in FIG. 16, a color LED chip 65 is disposed in a center part inside the LED unit 64.

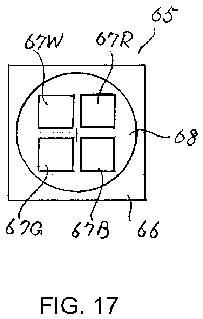

As shown in FIG. 17, in the color LED chip 65, an LED 67W which emits white (W), an LED 67R which emits red (R), an LED 67G which emits green (G), and an LED 67B which emits blue B are mounted on a ceramic substrate 66 and are coated with an encapsulating material 68 made of epoxy resin, silicone resin or the like.

Since the color LED chip 65 is disposed in the LED unit 64, the influence of chromatic aberration becomes conspicuous, and therefore it is preferable that the outer periphery of the LED unit 64 is surrounded by the reflector to control a ray of light.

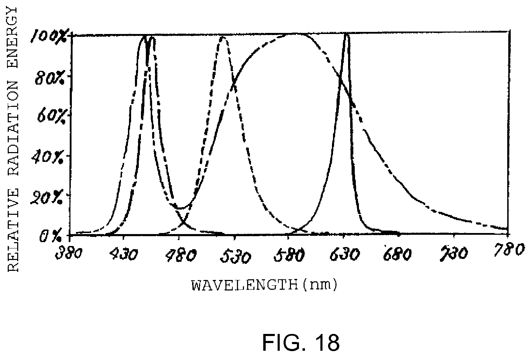

Here, the spectral characteristics of the LED 67W which emits white (W), the LED 67R which emits red (R), the LED 67G which emits green (G), and the LED 67B which emits blue (B) used in the present example are as shown in FIG. 18, and their luminous fluxes (lm) were as shown in Table 1.

In FIG. 18, the solid line shows the spectral characteristic of the LED 67R which emits red (R), the dotted line shows the spectral characteristic of the LED 67G which emits green (G), the alternate long and short dash line shows the spectral characteristic of the LED 67B which emits blue (B), and the two-dot chain line shows the spectral characteristic of the LED 67W which emits white (W).

Also, in Table 1, 4000K described in the column of LED 67W which emits white (W) indicates the color temperature.

**[Table 1]**

| Driving current | Luminous flux lm of LED (for each LED) | | | | |
|---|---|---|---|---|---|
| mA | White | Red | Green | Blue | Total |
| | 4000K | 625nm | 525nm | 460nm | |
| 350 | 100 | 87.4 | 114 | 26.85 | 328 |
| 175 | 50 | 44 | 57 | 13 | 164 |

If the color LED chip 65 is adopted, as shown in FIG. 16, the color LED chip 65 is disposed in the center part inside the LED unit 64, such that the LED 67W which emits white (W), the LED 67R which emits red (R), the LED 67G which emits green (G), and the LED 67B which emits blue (B) can be disposed in the substantially central part of the LED unit 64.

As a result, since the LED unit 64 can be made to be close to a point light source, it is possible to prevent color unevenness due to mixing of the four colors as much as possible and secure sufficient illuminance even by the LED having a small luminous flux.

In the present example, according to Table 1, when the LEDs of all colors are lit with a driving current of 175 mA, the LEDs of all colors can display a luminous flux of 1641 m in total.

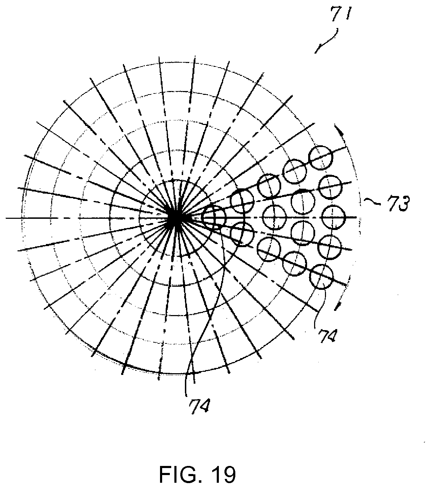

Next, as shown in FIG. 19, in an irradiation body 71 having a disk shape, the irradiation body 71 is constituted by irradiation units 73, 73,... which are divided into six parts in a circumferential direction and when the LED units 74, 74, are radially disposed from a center of the irradiation body 71, a focal position and illuminance thereat were examined.

Here, as shown in FIG. 19 and Table 2, the LED units 74, 74,... are radially disposed at a predetermined distance from the center of the irradiation body 71, and the LED units 74, 74,... in each stage are inclined inward so that the optical axes thereof face the center of the irradiation surface, and the inclination angle θ thereof is set so as to become larger toward the outer stage.

When the respective irradiation units 73, 73,... are in the horizontal state, that is, when the inclination angle θ from the horizon is set to be 0°, the bottom surface of the irradiation body 71 is set to be located at a position of 2.0 m from the floor surface.

**[Table 2]**

| | | Disposition diameter(mm) of LED | Disposition number of LEDs | Inclination angle θ of light source | Height Z(m) of light source |
|---|---|---|---|---|---|
| Inner | 1 | 150 | 6 | -2.8 | 2.022 |
| | 2 | 270 | 12 | -5.1 | 2018 |
| ↓ | 3 | 390 | 18 | -7.4 | 2011 |
| | 4 | 510 | 24 | -9.6 | 2003 |
| Outer | 5 | 630 | 30 | -11.9 | 1.991 |
| Total | | | 90 | | |

Furthermore, by appropriately changing the inclination angle θ from the horizon of the respective irradiation units 73, 73,..., the focal position of the light irradiated from the irradiation body 71 and the illuminance thereat were analyzed by simulation.

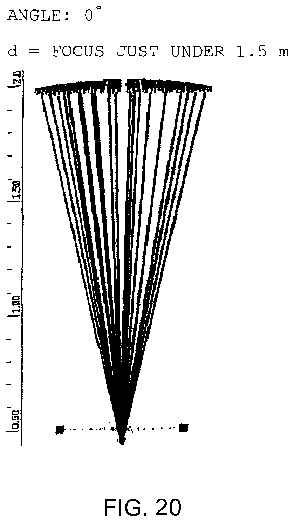

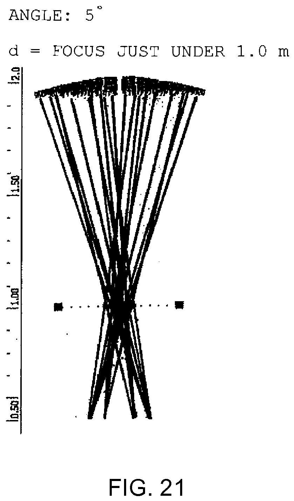

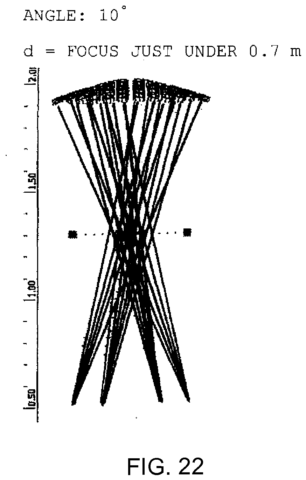

As a result, the focal position of the light irradiated from the irradiation body 71 when the inclination angle θ of the irradiation units 73, 73,... was appropriately changed was as shown in FIGS. 20 to 22.

In FIGS. 20 to 22, d indicates a distance from the bottom surface of the irradiation body 71 to a predetermined position thereunder.

As a result, since the mounting surface of the operating table on which a patient lies is usually positioned at a distance of about 1.0 m from the irradiation body 71, under the present implementing conditions, it was found that the inclination angle θ of the irradiation units 73, 73,... is suitably 5°.

In the case of the distance d (1.5 m, 1.0 m, 0.7 m) from the bottom surface of the irradiation body 71 when the inclination angle θ of the irradiation units 73, 73,... is appropriately changed, the illuminance at the appropriate position within the range of 250 mm in diameter around the central axis of the irradiation body 71 as the center was as shown in Tables 3 to 5.

As a result, since the maximum illuminance at the distance d of 1.0 m from the irradiation body 71 is 247, 3841x, under the present implementing conditions, it was found that it is possible to secure sufficient illuminance on the mounting surface of the operating table.

**[Table 3]**

| • Illumination at d = 1.5 m | | | |
|---|---|---|---|
| Mounting angle | Average | Minimum | Maximum |
| -5 | 41,929 | 39,927 | 43,612 |
| -4 | 54,277 | 47,417 | 57,488 |
| -3 | 66,317 | 51,566 | 78,798 |
| -2 | 76,445 | 53,203 | 102,706 |
| -1 | 83,772 | 53,554 | 127,338 |
| 0 | 87,557 | 53,365 | 145,245 |
| 1 | 87,440 | 53,093 | 144,981 |
| 2 | 83,419 | 53,000 | 126,715 |
| 3 | 75,881 | 52,514 | 102,578 |
| 4 | 65,530 | 50,381 | 78,857 |
| 5 | 53,325 | 46,110 | 57,494 |

**[Table 4]**

| • Illumination at d = 1.0 m | | | |
|---|---|---|---|
| Mounting angle | Average | Minimum | Maximum |
| 0 | 90,018 | 64,053 | 115,877 |
| 1 | 101,277 | 62,157 | 151,240 |
| 2 | 110,404 | 58,360 | 187,296 |
| 3 | 117,014 | 54,847 | 218,646 |
| 4 | 120,844 | 52,220 | 240,027 |
| 5 | 121,836 | 51,326 | 247,384 |
| 6 | 120,012 | 52,002 | 239,243 |
| 7 | 115,402 | 54,293 | 216,234 |
| 8 | 108,095 | 57,044 | 182,691 |
| 9 | 98,395 | 59,680 | 145,030 |
| 10 | 86,780 | 61,007 | 109,116 |

**[Table 5]**

| • Illumination at d = 0.7 m | | | |
|---|---|---|---|
| Mounting angle | Average | Minimum | Maximum |
| 5 | 111,259 | 65,354 | 188,284 |
| 6 | 119,598 | 62,503 | 212,617 |
| 7 | 126,524 | 59,068 | 234,842 |
| 8 | 131,711 | 55,959 | 250,799 |
| 9 | 135,207 | 53,640 | 262,663 |
| 10 | 137,144 | 50,728 | 270,349 |
| 11 | 137,292 | 49,438 | 272,599 |
| 12 | 135,689 | 49,931 | 266.504 |
| 13 | 132,245 | 51,910 | 255,003 |
| 14 | 127,305 | 54,760 | 235,704 |
| 15 | 121,116 - | 57,951 | 210,352 |

As described above, by adopting the configuration of the medical shadowless lamp of the present invention, since the light is condensed by changing the irradiation angle of the peripheral irradiation unit to sufficiently secure the illuminance of the light irradiated on the affected parts or the like, the surgery or the like can be easily performed.

In addition, since the irradiation angle of the peripheral irradiation unit is changed to be able to appropriately adjust the illuminance of light irradiated on the affected parts or the like during the surgery or treatment, the wasteful power consumption is prevented, such that energy can be saved.

In the above description, the ceiling-mounted type medical shadowless lamp has been described, but it goes without saying that the floor-mounted type medical shadowless lamp can also be applied.

In addition, although the irradiation body 71 is constituted by the plurality of irradiation units 73 disposed on the bottom surface of the circular housing 72, it is not limited to the circular shape, but the housing having various shapes such as a rectangular shape and a hexagonal shape can be selected.

## Claims

1. A medical shadowless lamp, wherein a plurality of irradiation units are disposed in an irradiation body, LED units are disposed in the irradiation units, and LED chips which emit three primary colors of red (R), green (G), and blue (B) are disposed in the LED units.

2. The medical shadowless lamp according to claim 1, wherein LEDs which emit three primary colors of red (R), green (G), and blue (B) are disposed in the LED units at appropriate intervals in a circumferential direction.

3. The medical shadowless lamp according to claim 1, wherein the LED chips which emit the three primary colors of red (R), green (G), and blue (B) and an LED chip which emits white (W) are simultaneously disposed in the LED units.

4. The medical shadowless lamp according to claim 1, wherein a color LED chip in which an LED which emits white (W), an LED which emits red (R), an LED which emits green (G), and an LED which emits blue (B) are integrated is disposed in the LED unit.

5. The medical shadowless lamp according to any one of claims 1 to 4, wherein the irradiation body is constituted by a central irradiation unit disposed at a central part thereof and a plurality of peripheral irradiation units disposed in a peripheral part thereof, and the peripheral irradiation unit is freely inclined with respect to the central irradiation unit.

6. The medical shadowless lamp according to claim 5, wherein the peripheral irradiation units are disposed at equal intervals around the central irradiation unit.

7. The medical shadowless lamp according to any one of claims 1 to 5, wherein the irradiation body is constituted by the plurality of irradiation units divided at equal intervals in a circumferential direction, and in each irradiation unit, the LED unit is radially disposed from a center.
